# EUROPÄISCHE PATENTSCHRIFT

(11) **EP 1 005 880 B1**
(45) Veröffentlichungstag und Bekanntmachung des Hinweises auf die Patenterteilung: **26.05.2004**
(21) Anmeldenummer: 99123950.0
(22) Anmeldetag: 03.12.1999
(51) Int. Cl.: A61N 5/06

(54) **Wärmetherapie-Behandlungsleuchte für den Handbetrieb**
Hand-held Thermotherapy lamp
Lampe à main de thermothérapie

(30) Priorität: 04.12.1998 DE 19856002
(43) Veröffentlichungstag der Anmeldung: 07.06.2000
(73) Patentinhaber: Ismail, Apul, 28211 Bremen (DE)
(72) Erfinder: Ismail, Apul, 28211 Bremen (DE)
(74) Vertreter: Goddar, Heinz J., Dr.

(56) Entgegenhaltungen:
- EP-A- 0 565 331
- EP-A- 0 815 824
- WO-A-96/04959
- DE-A- 3 347 730
- DE-U- 8 628 810
- FR-A- 617 966
- US-A- 4 658 823

## Beschreibung

Die vorliegende Erfindung betrifft eine Wärmetherapie-Behandlungsleuchte für den Handbetrieb.

Eine Infrarotlampe stellt eine klassische Wärmetherapie-Behandlungsleuchte dar. Die Infrarotlampe wird mit einer Spannung von 220 Volt betrieben und muß in einem relativ weiten Abstand vom Patienten angeordnet werden, da bei der Bestrahlung im sichtbaren Spektralbereich das Schmerzempfinden relativ früh eintritt. Der relativ weite Abstand sowie die Größe der Infrarotlampe führen zu einer recht großen Behandlungsfläche, so daß die Infrarotlampe nicht für eine gezielte Behandlung einer kleinen Fläche geeignet ist.

Aus der DE 33 47 730 A1 ist ein Wärmetherapie-Behandlungsgerät mit einer in einem Gehäuse mit Reflektor angeordneten Halogenlampe bekannt. Das Behandlungsgerät ermöglicht eine gezieltere Behandlung einer relativ kleinen Fläche. Die Dosis, d.h. die Energie der Strahlung pro Behandlungsfläche, läßt sich jedoch nur derart ungenau einstellen, daß das Wärmetherapie-Behandlungsgerät insbesondere nicht für eine Anwendung (Selbstmedikation) durch einen Patienten selbst geeignet ist. Eine genaue Dosierung der Wärmemenge ist jedoch zum einen zur Vermeidung von möglichen Verbrennungen und zum anderen zur geeigneten Therapierung der jeweiligen Beschwerden des Patienten erforderlich.

Dokument US-A-4 658 823 beschreibt ein wärmetherapie-Behandlungsleuchte gemäß dem Oberbegriff von Anspruch 1.

Der Erfindung liegt somit die Aufgabe zugrunde, ein Wärmetherapie-Behandlungsgerät bereitzustellen, mit dem eine leichte Dosierbarkeit der Wärmemenge ermöglicht wird. Darüber hinaus soll das Wärmetherapie-Behandlungsgerät im Handbetrieb einsetzbar sein.

Erfindungsgemäß wird diese Aufgabe gelöst durch eine Wärmetherapie-Behandlungsleuchte für den Handbetrieb mit einer elektrischen Lampe als Wärmetherapie-Strahlungsquelle, einem Gehäuse, das die elektrische Lampe umgibt und eine Gehäuseöffnung zum Austritt der Strahlung aufweist, einem mit dem Gehäuse einteilig ausgebildeten Handgriff, einer Stromversorgungseinrichtung für die Lampe, einem Distanzstück zur Einhaltung einer festgelegten Lichtstrecke zwischen der Lampe und Behandlungsfläche in Form eines Aufsatzes, dadurch gekennzeichnet, daß die Position des Aufsatzes relativ zur Lampe zumindest über einen Bereich entlang der Hauptlichtausbreitungsrichtung der Lampe zu der Behandlungsfläche variierbar ist und eine Schalteinrichtung zum automatischen Einschalten der Lampe beim Aufsetzen des Distanzstückes auf der Behandlungsfläche und automatischen Abschalten der Lampe bei nachfolgendem Entfernen des Distanzstückes von der Behandlungsfläche vorgesehen ist.

Weiterhin kann vorgesehen sein, daß ist die elektrische Lampe eine Halogen-Lampe mit einem Reflektor oder eine Niedervolt-Halogen-Lampe ist. Letztere vermeidet lebensgefährliche Stromschläge im Bereich des Gehäuses, sofern eine gegebenenfalls erforderliche Umwandlung einer 220 Volt-Spannung in eine Niederspannung relativ weit von dem Gehäuse entfernt stattfindet.

Vorzugsweise beträgt die Betriebsspannung der Niedervolt-Halogen-Lampe 12 Volt. Dies ist unter dem bereits in dem letzten Absatz genannten Sicherheitsaspekt besonders vorteilhaft.

Günstigerweise beträgt die Leistung der Lampe 20 Watt.

Gemäß einer besonderen Ausführungsform der Erfindung kann vorgesehen sein, daß die Niedervolt-Halogen-Lampe eine Halogen-Kaltlicht-Spiegellampe ist.

Günstigerweise weist die Halogen-Kaltlicht-Spiegellampe ein Sicherheitsglas auf.

Insbesondere kann dabei vorgesehen sein, daß das Sicherheitsglas eine Filtereinrichtung zur Filterung zumindest eines Teils des UVC-Anteils und/oder eine Filtereinrichtung zur Filterung zumindest eines Teils des UVB-Anteils und/oder eine Filtereinrichtung zur Filterung zumindest eines Teils des UVA-Anteils aufweist.

Vorteilhafterweise weist die Halogen-Lampe einen Abstrahlwinkel im Bereich von 10° bis 60° auf.

Vorzugsweise beträgt der Abstrahlwinkel 12°.

Günstigerweise weist der Reflektor auf der Strahlungsaustrittsseite einen Durchmesser im Bereich von 3,4 bis 9 cm auf.

Vorzugsweise beträgt der Durchmesser des Reflektors auf der Strahlungsaustrittsseite 5,1 cm.

Zweckmäßigerweise liegt die Temperatur des Glühfadens der Halogen-Lampe im Bereich von 2400 bis 2600 °C.

Vorzugsweise beträgt die Temperatur des Glühfadens 2500 °C.

Günstigerweise nimmt die Temperatur von der Mitte des Sicherheitsglases radial nach außen von ca. 80° C auf ca. 50° C ab.

Vorzugsweise beträgt die Temperatur in der Mitte des Sicherheitsglases 80 °C.

Eine weitere besondere Ausführungsform der Wärmetherapie-Behandlungsleuchte ist durch eine Fokussiereinrichtung zur Fokussierung der Strahlung der Lampe gekennzeichnet.

Dabei kann vorgesehen sein, daß die Fokussierwirkung der Fokussiereinrichtung variierbar ist. Dadurch läßt sich die Energie der Strahlung pro Behandlungsfläche variieren.

Darüber hinaus kann die Wärmetherapie-Behandlungsleuchte eine Ausblendeinrichtung zum Ausblenden eines Teils der Strahlung der Lampe aufweisen. Dadurch läßt sich die Größe der Behandlungsfläche beeinflussen.

Günstigerweise ist die Ausblendwirkung der Ausblendeinrichtung variierbar.

Dabei kann vorgesehen sein, daß der Aufsatz die Gestalt eines Ringes oder die Gestalt eines Trichters aufweist.

Insbesondere kann dabei vorgesehen sein, daß das weite Ende des Trichters der Behandlungsfläche oder der Lampe zugewandt ist.

Gemäß einer weiteren besonderen Ausführungsform der Erfindung ist der Aufsatz auswechselbar. Dies ermöglicht die Verwendung von Aufsätzen mit unterschiedlichen Abmessungen zur Variation des Abstandes zwischen der Lampe und der Behandlungsfläche.

Insbesondere kann dabei vorgesehen sein, daß die Position des Aufsatzes durch Verschieben oder durch eine Schraubbewegung veränderbar ist.

Vorteilhafterweise ist mittels des Distanzstückes der Abstand zwischen dem weiten Ende des Reflektors und der Behandlungsfläche im Bereich von 0,5 bis 4,5 cm variierbar.

Eine weitere besondere Ausführungsform der Erfindung ist gekennzeichnet durch eine Skaleneinrichtung zum Ablesen eines Maßes für den Abstand zwischen der Lampe und der Behandlungsfläche. Dies ermöglicht die Einstellung und Überprüfung eines bestimmten Abstandes zwischen der Lampe und der Behandlungsfläche.

Eine weitere besondere Ausführungsform der Erfindung ist gekennzeichnet durch eine Zeitschaltuhr (dies ermöglicht es, eine Behandlungsdauer vorzugeben) und/oder eine Temperaturmeßeinrichtung im Bereich der Lampe (dies ermöglicht eine Überwachung der Temperatur im Bereich der Lampe und ein Abschalten der Lampe bei einer möglichen Gefahr für den Patienten bzw. für die Lampe im Falle eines Wärmestaus) und/oder eine Kammer zur Aufnahme eines Duftstoffes oder einer Duftflüssigkeit und/oder Lüftungsschlitze im Gehäuse. Durch letztere wird ein Hitzestau vermieden.

Gemäß einer weiteren besonderen Ausführungsform der Erfindung kann vorgesehen sein, daß die Stromversorgungseinrichtung eine oder mehrere in dem Gehäuse untergebrachte Batterie(n) oder einen oder mehrere in dem Gehäuse untergebracht(n) Akku(s) umfaßt (ein Batterie- bzw. Akkubetrieb ermöglicht eine netzunabhängige Verwendung der Wännetherapie-Behandlungs-leuchte) und/oder eine in dem Gehäuse angeordnete Anschlußeinrichtung zum Anschluß der Lampe an eine Stromquelle umfaßt. Günstigerweise ist die Stromquelle eine 12 Volt-Stromquelle (dies verhindert zumindest im Bereich des Gehäuses einen lebensgefährlichen Stromschlag) oder eine 220 Volt-Stromquelle ist.

Dabei kann insbesondere vorgesehen sein, daß die Stromversorgungseinrichtung einen Transformator zur Umwandlung der 220 Volt-Spannung in eine 12 Volt-Spannung umfaßt.

Schließlich kann vorgesehen sein, daß sich der Transformator außerhalb des Gehäuses befindet. Wenn sich der Transformator z.B. im Bereich des Netzsteckers befindet und zwischen dem Transformator und dem Gehäuse der Wärmetherapie-Behandlungsleuchte ein relativ langes Stromkabel vorgesehen ist, so muß der Benutzer der Wärmetherapie-Behandlungsleuchte im Prinzip ein Niedervolt-Handgerät bedienen, so daß selbst Kinder die Wärmetherapie-Behandlungsleuchte bei entsprechender Anweisung bedienen können.

Der Erfindung liegt die überraschende Erkenntnis zugrunde, daß durch das Distanzstück eine leichte Dosierbarkeit der Wärmemenge, die durch die Energie der Strahlung pro Behandlungsfläche definiert ist, bereitgestellt. Das Distanzstück kann darüber hinaus den Zweck erfüllen, daß eine Verringerung der Dosis, d.h. der Energie der Strahlung pro Behandlungsfläche, nicht durch Inkaufnahme einer größeren Behandlungsfläche erzielt wird, sondern die Behandlungsfläche unverändert bleibt. Unter dem Sicherheitsaspekt ist insbesondere eine Ausführungsform mit einer Niedervolt-Halogen-Lampe sowie einem außerhalb des Gehäuses angeordneten Transformators besonders vorteilhaft. Die Wärmetherapie-Behandlungsleuchte ist daher für eine Selbstbehandlung durch Patienten besonders geeignet. Eine Selbstbehandlungsmöglichkeit erspart Arztbesuche bzw. den Einsatz von Personal bei der Durchführung der Wärmetherapie in einer Arztpraxis oder in einer Klinik.

Weitere Merkmale und Vorteile der Erfindung ergeben sich aus den Ansprüchen und aus der nachstehenden Beschreibung, in der ein Ausführungsbeispiel anhand der einzigen Zeichnung im einzelnen erläutert ist. Dabei zeigt:
Fig. 1 eine Schnittansicht einer Wärmetherapie-Behandlungsleuchte gemäß einer besonderen Ausführungsform der vorliegenden Erfindung;
Fig. 2 eine Schnittansicht wie Fig. 1, jedoch mit von der Wärmetherapie-Behandlungsleuchte getrenntem Distanzstück;
Fig. 3 eine Ansicht der Wärmetherapie-Behandlungsleuchte von Fig. 1 von unten;
Fig. 4 die Wärmetherapie-Behandlungsleuchte von Fig. 1 im Einsatz an verschiedenen Stellen des menschlichen Körpers;
Figur 5 eine Schnittansicht_einer Wärmetherapie-Behandlungsleuchte gemäß einer weiteren besonderen Ausführungsform der Erfindung in ausgeschaltetem Zustand;
Figur 6 die Wärmetherapie-Behandlungsleuchte von Figur 5 in eingeschaltetem Zustand;
Figur 7 die wesentlichen Komponenten der in Figur 5 gezeigten Wärmetherapie-Behandlungsleuchte;
Figur 8a eine Schnittansicht der Lampenhalterung der Wärmetherapie-Behandlungsleuchte von Figur 5; und
Figur 8b eine Draufsicht der Lampenhalterung von Figur 8a.

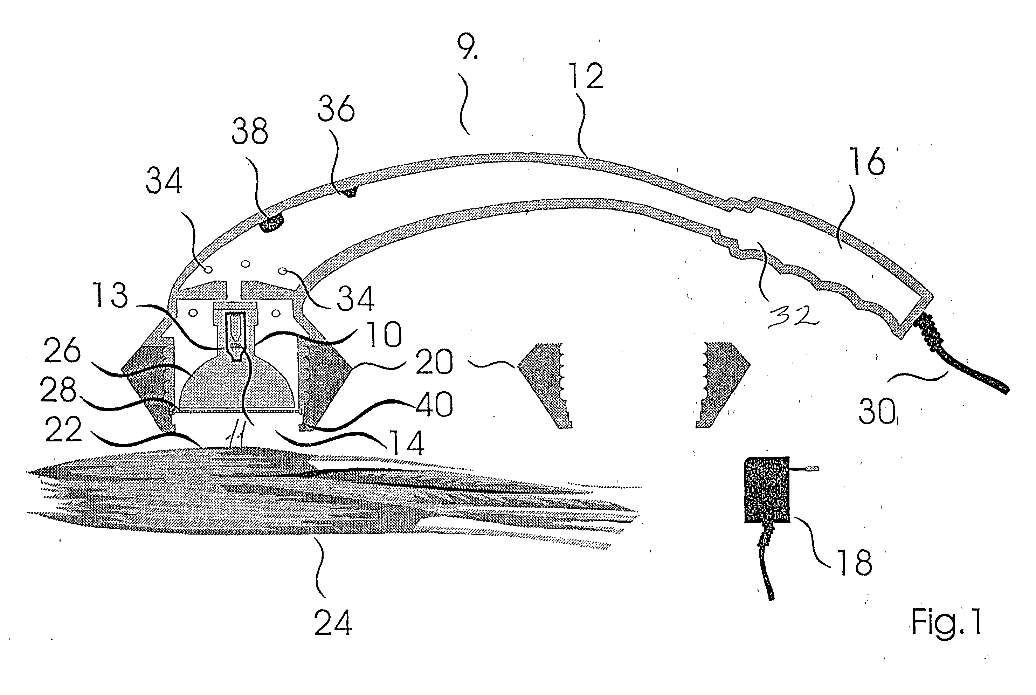

Figur 1 zeigt eine Schnittansicht einer Wärmetherapie-Behandlungsleuchte 9 gemäß einer besonderen Ausführungsform der vorliegenden Erfindung in Form einer Wätmetherapie-Behandlungsleuchte 9 für den Handbetrieb mit einer elektrischen Lampe 10 als Wärmetherapie-Strahlungsquelle, einem Gehäuse 12, das die elektrische Lampe 10 umgibt und eine Gehäuseöffnung 14 zum Austritt der Strahlung aufweist, einem mit dem Gehäuse 12 einteilig ausgebildeten Handgriff 16, einer Stromversorgungseinrichtung 18 für die Lampe 10 und einem Distanzstück 20 zur Einhaltung einer festgelegten Lichtstrecke zwischen der Lampe 10 und einer Behandlungsfläche 22 eines nur angedeuteten menschlichen Armes 24. Bei der Lampe 10 handelt es sich um eine 12 Volt-20 Watt-Halogen-Kaltlicht-Spiegellampe. Ein derartiger Halogen-Lampentyp weist einen Reflektor 26 auf. Darüber hinaus ist die Lampe 10 mit einem Sicherheitsglas 28 in Form einer Glasscheibe versehen. Die Temperatur des Glühfadens 11 der Lampe 10 beträgt 2500° C, während die Temperatur im Bereich des Kolbens 13 nur noch 300° C beträgt. Schließlich ist die Temperatur im Bereich des Sicherheitsglases 28 auf 80° C (Mitte) bzw. 50° C (Rand) abgefallen. Im Bereich des Handgriffes 16 tritt eine zu einer Stromversorgungseinrichtung 18 gehörige 12 Volt-Stromleitung 30 in das Gehäuse ein und erstreckt sich zur Versorgung der Lampe 10 mit Strom bis zur Lampe 10 (wobei dies nicht gezeigt ist). Neben der Stromleitung 30 weist die Stromversorgungseinrichtung 18 einen Transformator zur Umwandlung einer 220 Volt-Netzspannung in die 12 Volt-Niederspannung auf. Darüber hinaus befindet sich im Handgriff 16 eine Sicherung 32 zur Vermeidung von Stromschlägen. Im Bereich der Lampe 10 sind in dem Gehäuse 12 Lüftungslöcher 34 zur Ableitung von übermäßiger Wärme vorgesehen. Darüber hinaus wird von einer Temperaturmeßeinrichtung 36 in Form eines Minithermostats die Temperatur und deren Entwicklung im Bereich der Lampe 10 gemessen und überwacht, so daß die Lampe 10 bei einer übermäßigen Wärmeentwicklung automatisch abgeschaltet wird. Ebenfalls im Bereich der Lampe 10 befindet sich eine Kammer 38 zur Aufnahme einer Duftflüssigkeit, die langsam verdampft und einen angenehmen Duft verbreitet. Die Kammer 38 ermöglicht somit eine gleichzeitige Aromatherapie. Das Distanzstück 20 weist die Gestalt eines Ringes mit einem Innengewinde auf. Der Abstand zwischen dem Sicherheitsglas 28 und der Behandlungsfläche 22 kann durch Schrauben des Distanzstückes 20 in der entsprechenden Richtung über einen Bereich von 0,8 bis 3,9 cm variiert werden. Darüber hinaus ist in dem Distanzstück 20 eine Schalteinrichtung 40 zum automatischen Einschalten der Lampe 10 beim Aufsetzen des Distanzstückes 20 auf der Behandlungsfläche 22 und automatischen Abschalten der Lampe 10 beim nachfolgendem Entfernen des Distanzstückes 20 von der Behandlungsfläche 22 integriert. Alternativ oder in Ergänzung zu der Schalteinrichtung 40 kann eine Zeitschaltuhr vorgesehen sein.

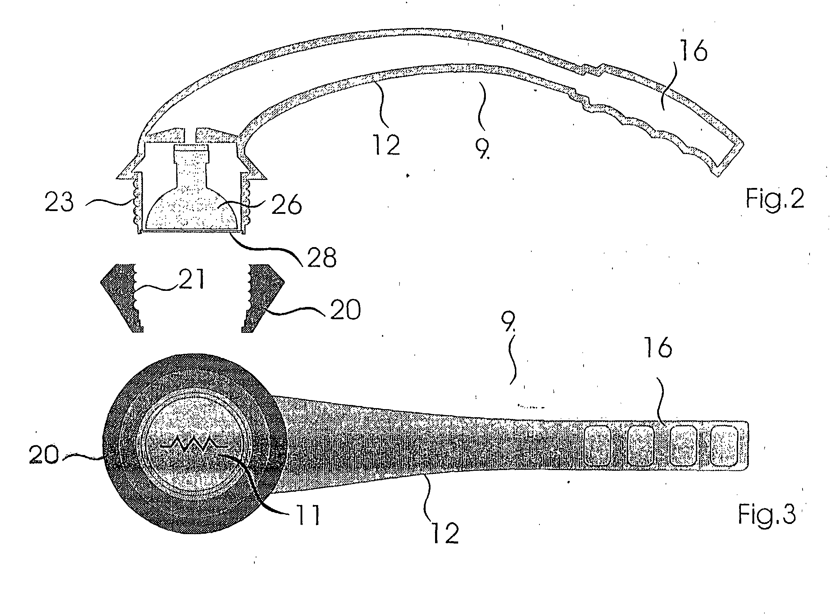

Fig. 2 zeigt eine Schnittansicht wie Fig. 1, jedoch mit von der Wärmetherapie-Behandlungsleuchte 9 getrenntem Distanzstück 20. Das Distanzstück 20 weist ein Innengewinde 21 auf, das mit einem Außengewinde 23 des Gehäuses 12 in Eingriff bringbar ist, so daß das Distanzstück 20 sicher an dem Gehäuse 12 gehalten wird und der Abstand zwischen dem Sicherheitsglas 28 und einer Behandlungsfläche durch Hinein- bzw. Herausschrauben variierbar ist.

Fig. 3 zeigt eine Ansicht der Wärmetherapie-Behandlungsleuchte 9 von Fig. 1 von unten.

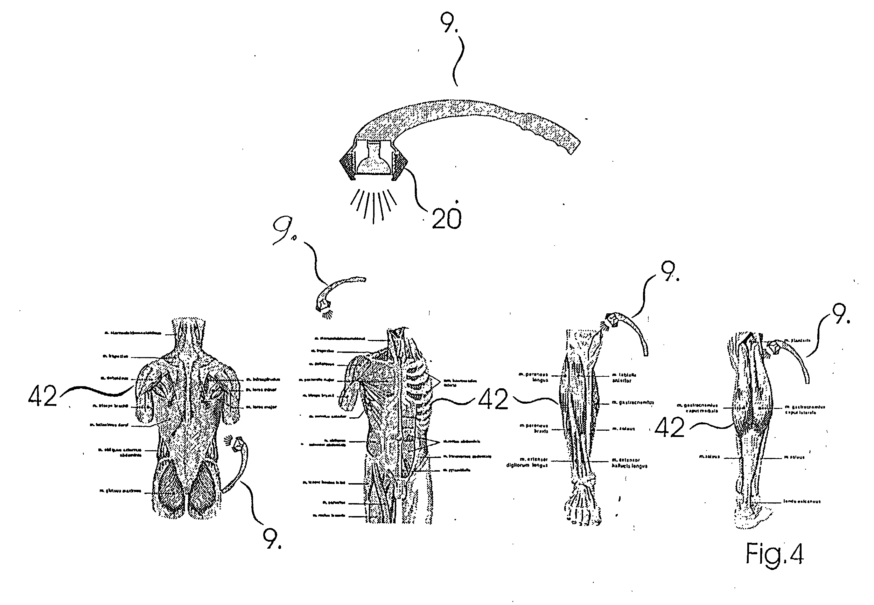

Fig. 4 zeigt die Wärmetherapie-Behandlungsleuchte 9 von Fig. 1 im Einsatz an verschiedenen Stellen des menschlichen Körpers 42. Die Wärmetherapie-Behandlungsleuchte 9 kann z.B. im Lendenbereich (Abbildung ganz links), Schulterbereich (zweite Abbildung von links), Kniebereich (zweite Abbildung von rechts) und Wadenbereich (Abbildung rechts) eingesetzt werden.

Die Wärmetherapie-Behandlungsleuchte wird wie folgt eingesetzt: Die Wärmetherapie-Behandlungsleuchte 9 wird mit dem Distanzstück 20 z.B. im Bereich der Wade auf das Bein eines Patienten gesetzt, wobei der Druck auf die Haut derart sein sollte, daß die die Behandlungsfläche umgebende Fläche etwas rötliche Farbe bekommt. Beim Berühren der Haut durch das Distanzstück 20 wird die Wärmetherapie-Behandlungsleuchte 9 mittels der Schalteinrichtung 40 automatisch eingeschaltet. Nach Verspüren eines Schmerzes, was üblicherweise in Abhängigkeit von der Behandlungsfläche und dem Schmerzempfinden des Patienten nach ca. 5 bis 20 Sekunden eintritt, wird die Wärmetherapie-Behandlungsleuchte 9 für ca. 2 bis 5 Sekunden von der Haut entfernt, während derer die Wärmetherapie-Behandlungsleuchte 9 durch die Schalteinrichtung 40 automatisch ausgeschaltet ist. Danach wird die Wärmetherapie-Behandlungsleuchte 9 mit dem Distanzstück 20 noch einmal für ca. 15 bis 20 Sekunden auf die Haut gesetzt. Dies wird nach einer weiteren Unterbrechung von 2 bis 5 Sekunden noch einmal wiederholt.

Die dreimalige Anwendung der Wärmetherapie-Behandlungsleuchte führt zu einer Erhöhung der Körpertemperatur von ca. 1,5 °C. Der Patient empfindet eine nadelstichartige Hitzefokussierung, die schon bald nicht mehr von der Haut toleriert wird. Die Nadelstichwirkung kann mit derjenigen einer chinesischen Akupunkturnadel verglichen werden, wobei dies mit der erfindungsgemäßen Wärmetherapie-Behandlungsleuchte ohne eine Nadel geschieht. Mit der erfindungsgemäßen Wärmetherapie-Behandlungsleuchte werden subkutan Thermorezeptoren arealweise stimuliert. Die Stimulierung der Nervenenden führt zu einer Kutiviszeral-Reflex-Zone mit einer vermehrten Ausschüttung von Serotonin, Endorphin, Melatonin.

Mit z.B. einer 20W-Lichtquelle ermöglicht das variierbare Distanzstück die Emission von 20 Photonen/Sekunde auf Körperzellen, wobei jedoch in Abhängigkeit von der jeweiligen Schmerzgrenze eines Patienten auch eine von der optimalen abweichende Distanz eingestellt werden kann.

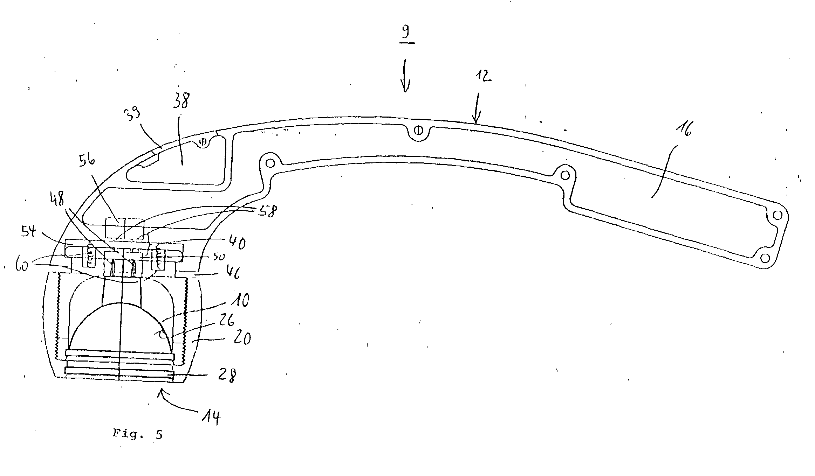

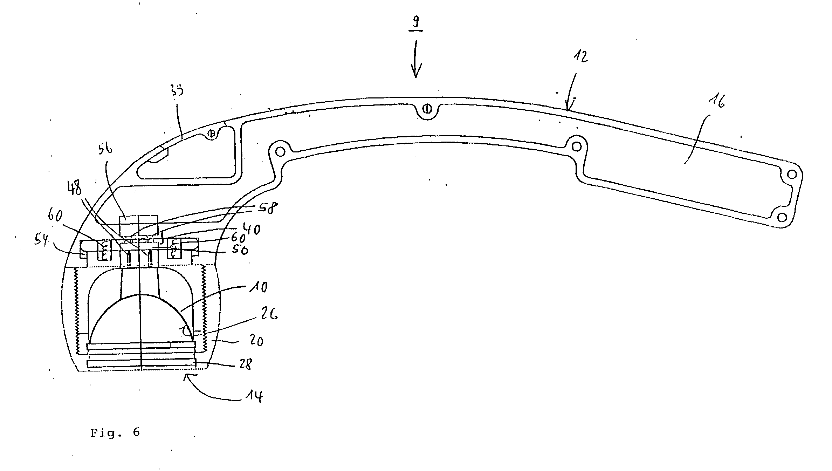

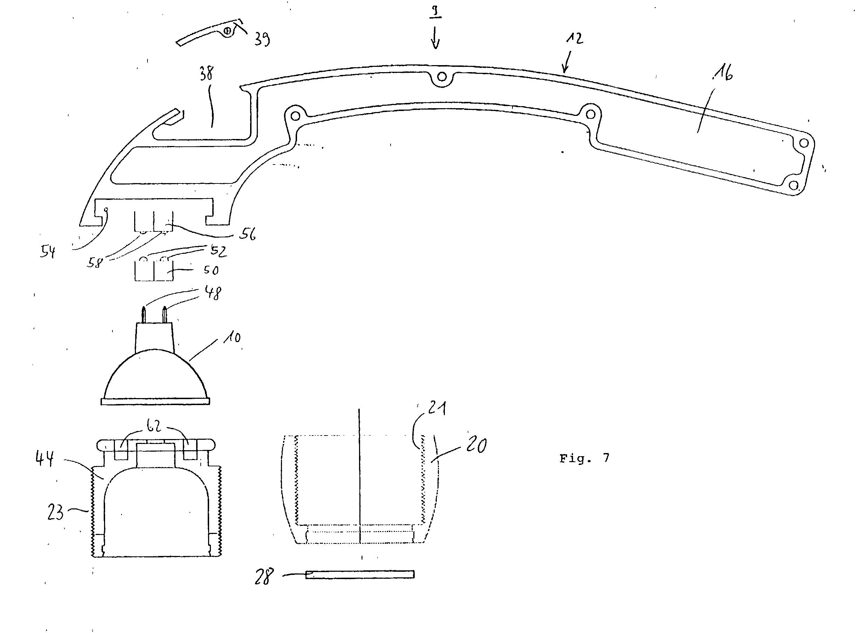

Figuren 5 und 6 zeigen jeweils eine Schnittansicht einer Wärmetherapie-Behandlungsleuchte gemäß einer weiteren besonderen Ausführungsform der Erfindung, wobei die Wärmetherapie-Behandlungsleuchte sich in Figur 5 im ausgeschalteten und in Figur 6 im eingeschalteten Zustand befindet. Nachfolgend soll im wesentlichen nur auf die Besonderheiten gegenüber der in Figur 1 gezeigten Wärmetherapie-Behandlungsleuchte eingegangen werden. Eine Lampe 10 befindet sich in einer verdrehsicheren Lampenhalterung 44. Die Lampenhalterung 44 weist ein Außengewinde auf, auf das ein Distanzstück 20 mit einem Innengewinde 21 geschraubt ist. Der Abstand zwischen der Lampe und einer Behandlungsfläche kann durch Hinein- bzw. Herausschrauben des Distanzstückes 20 variiert werden. Weiterhin ist zwischen der Kombination des Distanzstückes und der Lampenhalterung und dem Gehäuse 12 im ausgeschalteten Zustand der Lampe 10 ein Ringspalt 46 vorgesehen. Die Stifte 48 der Lampe 10 befinden sich in einem Sockel 50 aus Keramik. Im hinteren Bereich des Sockels 50 befinden sich zwei elektrische Kontakte 52, die mit den Stiften 48 in Verbindung stehen (siehe auch Figur 7). Oberhalb einer Aussparung 54 in dem Gehäuse 12 befindet sich ein weiterer Sockel 56 aus Keramik mit elektrischen Kontakten 58, die den elektrischen Kontakten 52 zugewandt sind. Im ausgeschalteten Zustand werden die elektrischen Kontakte 52 und 58 durch Federn 60 voneinander getrennt. Wenn jedoch die Wärmetherapie-Behandlungsleuchte auf eine Behandlungsfläche (nicht gezeigt) aufgesetzt wird, werden die Federn 60 zusammengepreßt und entsteht ein elektrischer Kontakt zwischen den elektrischen Kontakten 52 und 58. Da die elektrischen Kontakte 58 mit einer Stromversorgung (nicht gezeigt) verbunden sind, wird somit die Lampe 10 eingeschaltet und bei Entfernen der Wärmetherapie-Behandlungsleuchte von der Behandlungsfläche ausgeschaltet.

Die Kammer 38 weist einen Deckel 39 auf.

Figur 7 zeigt noch einmal die wesentlichen Komponenten der Wärmetherapie-Behandlungsleuchte von Figur 5.

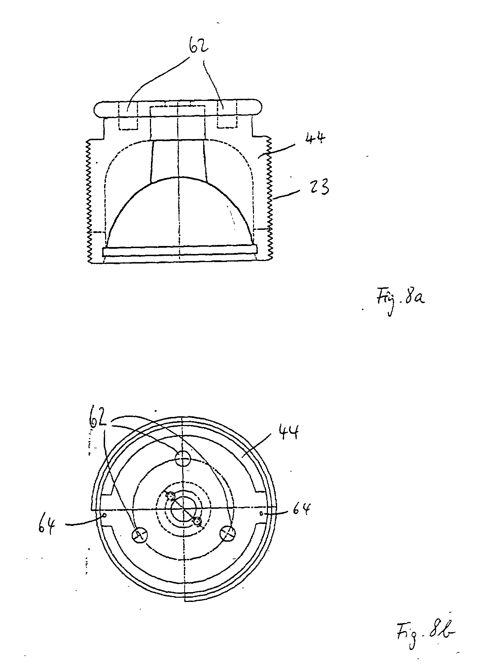

Figur 8a zeigt noch einmal eine Schnittansicht der Lampenhalterung 44.

Figur 8b zeigt eine Draufsicht der in Figur 8a gezeigten Lampenhalterung 44. Die Lampenhalterung 44 weist drei Vertiefungen 62 zur Aufnahme der Federn (nicht gezeigt) auf. Des weiteren weist sie zwei Kerben 64 zum verdrehsicheren Befestigen der Lampenhalterung 44 in der Aussparung 54 des Gehäuses 12 auf. Die in den Figuren 5 bis 8b gezeigte Konstruktion ermöglicht auf besonders elegante Weise eine Kombination eines variierbaren Distanzstückes mit einer Schalteinrichtung.

Die erfindungsgemäße Wärmetherapie-Behandlungsleuchte kann zur therapeutischen Behandlung des menschlichen Körpers bei Befindlichkeitsstörungen, Depressionen aller Art, bei Schlafstörungen, Angstzuständen, Panik und leichteren mittelgradigen Schmerzzuständen eingesetzt werden. Bei den Schmerzen kann es sich z.B. um Arthritis, Muskelschmerzen, Neuralgie handeln.

Die in der vorstehenden Beschreibung sowie in den Ansprüchen und der beiligenden Zeichnung offenbarten Merkmalen der Erfindung können sowohl einzeln als auch in beliebiger Kombination für die Verwirklichung der Erfindung in ihren verschiedenen Ausführungsformen wesentlich sein.

### Bezugszeichenliste

- 9: Wärmetherapie-Behandlungsleuchte
- 10: Lampe
- 11: Glühfaden
- 12: Gehäuse
- 13: Kolben
- 14: Gehäuseöffnung
- 16: Handgriff
- 18: Stromversorgungseinrichtung
- 20: Distanzstück
- 21: Innengewinde
- 22: Behandlungsfläche
- 23: Außengewinde
- 24: Arm
- 26: Reflektor
- 28: Sicherheitsglas
- 30: Stromleitung
- 32: Sicherung
- 34: Lüftungsloch
- 36: Temperaturmeßeinrichtung
- 38: Kammer
- 40: Schalteinrichtung
- 42: menschlicher Körper
- 44: Lampenhalterung
- 46: Ringspalt
- 48: Stift
- 50: Sockel
- 52: elektrischer Kontakt
- 54: Aussparung
- 56: Sockel
- 60: Federn
- 62: Vertiefung
- 64: Kerbe

## Patentansprüche

1. Wärmetherapie-Behandlungsleuchte für den Handbetrieb, mit
- einer elektrischen Lampe (10) als Wärmetherapie-Strahlungsquelle,
- einem Gehäuse (12), das die elektrische Lampe (10) umgibt und eine Gehäuseöffnung (14) zum Austritt der Strahlung aufweist,
- einem mit dem Gehäuse (12) einteilig ausgebildeten Handgriff (16),
- einer Stromversorgungseinrichtung (18) für die Lampe (10),
- einem Distanzstück (20) zur Einhaltung einer festgelegten Lichtstrecke zwischen der Lampe (10) und einer Behandlungsfläche (22) in Form eines Aufsatzes,
**dadurch gekennzeichnet, daß**
die Position des Aufsatzes relativ zur Lampe (10) zumindest über einen Bereich entlang der Hauptlichtausbreitungsrichtung der Lampe (10) zu der Behandlungsfläche (22) variierbar ist und
- eine Schalteinrichtung (40) zum automatischen Einschalten der Lampe (10) beim Aufsetzen des Distanzstückes (20) auf der Behandlungsfläche (22) und automatischen Abschalten der Lampe (10) bei nachfolgendem Entfernen des Distanzstückes (20) von der Behandlungsfläche (22) vorgesehen ist.

2. Wärmetherapie-Behandlungsleuchte nach Anspruch 1, **dadurch gekennzeichnet, daß** die elektrische Lampe (10) eine Halogen-Lampe mit einem Reflektor (26) oder eine Niedervolt-Halogen-Lampe ist.

3. Wärmetherapie-Behandlungsleuchte nach Anspruch 2, **dadurch gekennzeichnet, daß** die Betriebsspannung der Niedervolt-Halogen-Lampe 12 Volt beträgt.

4. Wärmetherapie-Behandlungsleuchte nach Anspruch 2, **dadurch gekennzeichnet, daß** die Leistung der Lampe (10) 20 Watt beträgt.

5. Wärmetherapie-Behandlungsleuchte nach Anspruch 3 oder 4, **dadurch gekennzeichnet, daß** die Niedervolt-Halogen-Lampe eine Halogen-Kaltlicht-Spiegellampe ist.

6. Wärmetherapie-Behandlungsleuchte nach Anspruch 5, **gekennzeichnet durch** ein Sicherheitsglas (28).

7. Wärmetherapie-Behandlungsleuchte nach Anspruch 6, **dadurch gekennzeichnet, daß** das Sicherheitsglas (28) eine Filtereinrichtung zur Filterung zumindest eines Teils des UVC-Anteils und/oder eine Filtereinrichtung zur Filterung zumindest eines Teils des UVB-Anteils und/oder eine Filteranordnung zur Filterung zumindest eines Teils des UVA-Anteils aufweist.

8. Wärmetherapie-Behandlungsleuchte nach einem der Ansprüche 2 bis 7, **dadurch gekennzeichnet, daß** die Halogen-Lampe einen Abstrahlwinkel im Bereich von 10° bis 60 ° aufweist.

9. Wärmetherapie-Behandlungsleuchte nach Anspruch 8, **dadurch gekennzeichnet, daß** der Abstrahlwinkel 12 ° beträgt.

10. Wärmetherapie-Behandlungsleuchte nach einem der Ansprüche 2 bis 9, **dadurch gekennzeichnet, daß** der Reflektor (26) auf der Strahlungsaustrittsseite einen Durchmesser im Bereich von 3,4 bis 9 cm aufweist.

11. Wärmetherapie-Behandlungsleuchte nach Anspruch 10, **dadurch gekennzeichnet, daß** der Durchmesser des Reflektors (26) auf der Strahlungsaustrittsseite 5,1 cm beträgt.

12. Wärmetherapie-Behandlungsleuchte nach einem der Ansprüche 2 bis 11, **dadurch gekennzeichnet, daß** die Temperatur des Glühfadens der Halogen-Lampe im Bereich von 2400 bis 2600 °C liegt.

13. Wärmetherapie-Behandlungsleuchte nach Anspruch 12, **dadurch gekennzeichnet, daß** die Temperatur des Glühfadens 2500 °C beträgt.

14. Wärmetherapie-Behandlungsleuchte nach einem der Ansprüche 6 bis 13, **dadurch gekennzeichnet, daß** die Temperatur von der Mitte des Sicherheitsglases (28) radial nach außen von ca. 80 ° C auf ca. 50 ° C abnimmt.

15. Wärmetherapie-Behandlungsleuchte nach Anspruch 14, **dadurch gekennzeichnet, daß** die Temperatur in der Mitte des Sicherheitsglases (28) 80 ° C beträgt.

16. Wärmetherapie-Behandlungsleuchte nach einem der vorangehenden Ansprüche, **gekennzeichnet durch** eine Fokussiereinrichtung zur Fokussierung der Strahlung der Lampe (10).

17. Wärmetherapie-Behandlungsleuchte nach Anspruch 16, **dadurch gekennzeichnet, daß** die Fokussierwirkung der Fokussiereinrichtung variierbar ist.

18. Wärmetherapie-Behandlungsleuchte nach einem der vorangehenden Ansprüche, **gekennzeichnet durch** eine Ausblendeinrichtung zum Ausblenden eines Teils der Strahlung der Lampe (10).

19. Wärmetherapie-Behandlungsleuchte nach Anspruch 18, **dadurch gekennzeichnet, daß** die Ausblendwirkung der Ausblendeinrichtung variierbar ist.

20. Wärmetherapie-Behandlungsleuchte nach einem der vorangehenden Ansprüche, **dadurch gekennzeichnet, daß** der Aufsatz die Gestalt eines Ringes oder die Gestalt eines Trichters aufweist.

21. Wärmetherapie-Behandlungsleuchte nach Anspruch 20, **dadurch gekennzeichnet, daß** das weite Ende des Trichters der Behandlungsfläche (22) oder der Lampe (10) zugewandt ist.

22. Wärmetherapie-Behandlungsleuchte nach einem der vorangehenden Ansprüche, **dadurch gekennzeichnet, daß** der Aufsatz auswechselbar ist.

23. Wärmetherapie-Behandlungsleuchte nach einem der vorangehenden Ansprüche, **dadurch gekennzeichnet, daß** die Position des Aufsatzes durch Verschieben oder durch eine Schraubbewegung veränderbar ist.

24. Wärmetherapie-Behandlungsleuchte nach einem der Ansprüche 2 bis 23, **dadurch gekennzeichnet, daß** mittels des Distanzstückes (20) der Abstand zwischen dem weiten Ende des Reflektors (26) und der Behandlungsfläche (22) im Bereich von 0,5 bis 4,5 cm variierbar ist.

25. Wärmetherapie-Behandlungsleuchte nach einem der vorangehenden Ansprüche, **gekennzeichnet durch** eine Skaleneinrichtung zum Ablesen eines Maßes für den Abstand zwischen der Lampe (10) und der Behandlungsfläche (22).

26. Wärmetherapie-Behandlungsleuchte nach einem der vorangehenden Ansprüche, **gekennzeichnet durch** eine Zeitschaltuhr und/oder eine Temperaturmeßeinrichtung (36) im Bereich der Lampe (10) und/oder eine Kammer (38) zur Aufnahme eines Duftstoffes oder einer Duftflüssigkeit und/oder Lüftungsschlitze (34) in dem Gehäuse (12).

27. Wärmetherapie-Behandlungsleuchte nach einem der vorangehenden Ansprüche, **dadurch gekennzeichnet, daß** die Stromversorgungseinrichtung (18) eine oder mehrere in dem Gehäuse untergebrachte Batterie(n) oder einen oder mehrere in dem Gehäuse untergebrachte(n) Akku(s) und/oder eine in dem Gehäuse (12) angeordnete Anschlußeinrichtung zum Anschluß der Lampe (10) an eine Stromquelle umfaßt.

28. Wärmetherapie-Behandlungsleuchte nach Anspruch 27, **dadurch gekennzeichnet, daß** die Stromquelle eine 12 Volt-Stormquelle oder eine 220 Volt-Stromquelle ist.

29. Wärmetherapie-Behandlungsleuchte nach Anspruch 28, **dadurch gekennzeichnet, daß** die Stromversorgungseinrichtung (18) einen Transformator zur Umwandlung der 220 Volt-Spannung in eine 12 Volt-Spannung umfaßt.

30. Wärmetherapie-Behandlungsleuchte nach Anspruch 29, **dadurch gekennzeichnet, daß** sich der Transformator außerhalb des Gehäuses (12) befindet.

## Claims

1. A luminaire for heat therapy treatment, for manual operation, with
- an electric lamp (10) as the heat therapy radiation source,
- a housing (12) which surrounds the electric lamp (10) and has an opening (14) for the exit of the radiation,
- a handle (16) made in one piece with the housing (12),
- a power supply unit (18) for the lamp (10),
- a spacer (20) for maintaining a fixed lighting distance between the lamp (10) and a treatment surface (22), in the form of an attachment,
**characterised in that**
- the position of the attachment relative to the lamp (10) is variable at least over one zone along the main direction of propagation of the light from the lamp (10) to the treatment surface (22) and
- a switching device (40) for automatically switching the lamp (10) on when the spacer (20) is placed on the treatment surface (22) and automatically switching the lamp (10) off on subsequent removal of the spacer (20) from the treatment surface (22).

2. A heat therapy treatment luminaire according to claim 1, **characterised in that** the electric lamp (10) is a halogen lamp with a reflector (26) or a low-voltage halogen lamp.

3. A heat therapy treatment luminaire according to claim 2, **characterised in that** the operating voltage of the low-voltage halogen lamp is 12 volts.

4. A heat therapy treatment luminaire according to claim 2, **characterised in that** the power of the lamp (10) is 20 watts.

5. A heat therapy treatment luminaire according to claim 3 or 4, **characterised in that** the low voltage halogen lamp is a halogen cold-light reflector lamp.

6. A heat therapy treatment luminaire according to claim 5, **characterised by** a safety glass (28).

7. A heat therapy treatment luminaire according to claim 6, **characterised in that** the safety glass (28) has a filter device for filtering at least part of the UVC component and/or a filter device for filtering at least a part of the UVB component and/or filter arrangement for filtering at least a part of the UVA component.

8. A heat therapy treatment luminaire according to any one of claims 2 to 7, **characterised in that** the halogen lamp has a radiation emitting angle in the range from 10 to 60°.

9. A heat therapy treatment luminaire according to claim 8, **characterised in that** the radiation emitting angle is 12°.

10. A heat therapy treatment luminaire according to any one of claims 2 to 9, **characterised in that** the reflector (26) on the radiation exit side has a diameter in the range from 3.4 to 9 cm.

11. A heat therapy treatment luminaire according to claim 10, **characterised in that** the diameter of the reflector (26) on the radiation exit side is 5.1 cm.

12. A heat therapy treatment luminaire according to any one of claims 2 to 11, **characterised in that** the temperature of the filament of the halogen lamp is in the range from 2400 to 2600°C.

13. A heat therapy treatment luminaire according to claim 12, **characterised in that** the temperature of the filament is 2500°C.

14. A heat therapy treatment luminaire according to any one of claims 6 to 13, **characterised in that** the temperature decreases radially outwards from the centre of the safety glass (28) from about 80°C to about 50°C.

15. A heat therapy treatment luminaire according to claim 14, **characterised in that** the temperature in the centre of the safety glass (28) is 80°C.

16. A heat therapy treatment luminaire according to any one of the preceding claims, **characterised by** a focusing device for focusing the radiation of the lamp (10).

17. A heat therapy treatment luminaire according to claim 16, **characterised in that** the focusing action of the focusing device is variable.

18. A heat therapy treatment luminaire according to any one of the preceding claims, **characterised by** a screening device for screening out part of the radiation of the lamp (10).

19. A heat therapy treatment luminaire according to claim 18, **characterised in that** the screening effect of the screening device is variable.

20. A heat therapy treatment luminaire according to any one of the preceding claims, **characterised in that** the attachment has the form of a ring or the form of a funnel.

21. A heat therapy treatment luminaire according to claim 20, **characterised in that** the wide end of the funnel faces the treatment surface (22) or the lamp (10).

22. A heat therapy treatment luminaire according to any one of the preceding claims, **characterised in that** the attachment is interchangeable.

23. A heat therapy treatment luminaire according to any one of the preceding claims, **characterised in that** the position of the attachment is variable by sliding or by a screwing movement.

24. A heat therapy treatment luminaire according to any one of claims 2 to 23, **characterised in that** the distance between the wide end of the reflector (26) and the treatment surface (22) is variable in the range from 0.5 to 4.5 cm by means of the spacer (20).

25. A heat therapy treatment luminaire according to any one of the preceding claims, **characterised by** a scale device for reading off a measurement of the distance between the lamp (10) and the treatment surface (22).

26. A heat therapy treatment luminaire according to any one of the preceding claims, **characterised by** a time switch and/or a temperature measuring device (36) in the region of the lamp (10) and/or a chamber (38) to accommodate an aromatic substance or an aromatic liquid and/or venting slots (34) in the housing (12).

27. A heat therapy treatment luminaire according to any one of the preceding claims, **characterised in that** the power supply unit (18) comprises one or more batteries accommodated in the housing or one or more accumulators accommodated in the housing and/or a connection device disposed in the housing (12) for connecting the lamp (10) to a power supply.

28. A heat therapy treatment luminaire according to claim 27, **characterised in that** the power supply is a 12 volt power supply or a 220 volt power supply.

29. A heat therapy treatment luminaire according to claim 28, **characterised in that** the power supply unit (18) comprises a transformer for converting the 220 volt supply to a 12 volt supply.

30. A heat therapy treatment luminaire according to claim 29, **characterised in that** the transformer is situated outside the housing (12).

## Revendications

1. Luminaire de traitement de thermothérapie pour utilisation manuelle, comprenant :
- une lampe électrique (10), servant de source de rayonnement de thermothérapie,
- un boîtier (12), entourant la lampe électrique (10) et présentant une ouverture de boîtier (14) pour la sortie du rayonnement,
- une poignée (16), réalisée en une partie avec le boîtier (12),
- un dispositif d'alimentation électrique (18) pour la lampe (10)
- une pièce d'espacement (20), pour respecter une longueur du chemin fixée du chemin suivi par la lumière, entre la lampe (10) et une surface de traitement (22) ayant la forme d'une pièce rapportée,
**caractérisé en ce que**
la position de la pièce rapportée par rapport à la lampe (10) est modifiable sur une zone, le long de la direction de propagation principale de la lumière. de la lampe (10) vers la surface de traitement (22), et
- un dispositif de commutation (40), devant commuter automatiquement à la lampe (10) lors de la pose de la pièce d'espacement (20) sur la surface de traitement (22) et mettre automatiquement hors-service la lampe (10), lors de l'enlèvement subséquent de la pièce d'espacement (20) vis-à-vis de la surface de traitement (22), étant prévu.

2. Luminaire de traitement de thermothérapie selon la revendication 1, **caractérisé en ce que** la lampe électrique (10) est une lampe à halogène avec un réflecteur (26) ou bien une lampe à halogène basse tension.

3. Luminaire de traitement de thermothérapie selon la revendication 2, **caractérisé en ce que** la tension de fonctionnement de la lampe à halogène basse tension est de 12 Volts.

4. Luminaire de traitement de thermothérapies selon la revendication 2, **caractérisé en ce que** la puissance de la lampe (10) est de 20 Watts.

5. Luminaire de traitement de thermothérapie selon la revendication 3 ou 4, **caractérisé en ce que** la lampe halogène basse tension est une lampe à miroir à lumière froide, à halogène.

6. Luminaire de traitement de thermothérapie selon la revendication 5, **caractérisé par** un verre de sécurité (28).

7. Luminaire de traitement de thermothérapie selon la revendication 6, **caractérisé en ce que** le verre de sécurité (28) présente un dispositif de filtrage, pour filtrer au moins une partie de la fraction UVC, et/ou un dispositif de filtrage, pour filtrer au moins une partie de la fraction UVB, et/ou un dispositif de filtrage, pour filtrer au moins une partie de la fraction UVA.

8. Luminaire de traitement de thermothérapie selon l'une des revendications 2 à 7, **caractérisé en ce que** la lampe à halogène présente un angle d'irradiation dans la plage de 10° à 60°.

9. Luminaire de traitement de thermothérapie selon la revendication 8, **caractérisé en ce que** l'angle d'irradiation est de 12°.

10. Luminaire de traitement de thermothérapie selon l'une des revendications 2 à 9, **caractérisé en ce que** le réflecteur (26) présente, sur le côté sortie du rayonnement, un diamètre dans la plage de 3,4 à 9 cm.

11. Luminaire de traitement de thermothérapie selon la revendication 10, **caractérisé en ce que** le diamètre du réflecteur (26), sur le côté de sortie du rayonnement, est de 5,1 cm.

12. Luminaire de traitement de thermothérapie selon l'une des revendications 2 à 11, **caractérisé en ce que** la température du fil incandescent de la lampe à halogène est dans la plage de 2400 à 2600 °C.

13. Luminaire de traitement de thermothérapie selon la revendication 12, **caractérisé en ce que** la température du fil incandescent est de 2500 °C.

14. Luminaire de traitement de thermothérapie selon l'une des revendications 6 à 13, **caractérisé en ce que** la température va en diminuant vers l'extérieur depuis le centre du verre de sécurité (28), en passant d'environ 80° C à environ 50° C.

15. Luminaire de traitement de thermothérapie selon la revendication 14, **caractérisé en ce que** la température au centre du verre de sécurité (28) est de 80° C.

16. Luminaire de traitement de thermothérapie selon l'une des revendications précédentes, **caractérisé par** un dispositif de focalisation, devant focaliser le rayonnement de la lampe (10).

17. Luminaire de traitement de thermothérapie selon la revendication 16, **caractérisé en ce que** l'effet de focalisation du dispositif de focalisation est variable.

18. Luminaire de traitement de thermothérapie selon l'une des revendications précédentes, **caractérisé par** un dispositif occultation, pour occulter une partie du rayonnement de la lampe (10).

19. Luminaire de traitement de thermothérapie selon la revendication 18, **caractérisé en ce que** l'effet occultateur du dispositif occultateur est variable.

20. Luminaire de traitement de thermothérapie selon l'une des revendications précédentes, **caractérisé en ce que** la pièce rapportée présente la forme d'une bague ou la forme d'un entonnoir.

21. Luminaire de traitement de thermothérapie selon la revendication 20, **caractérisé en ce que** l'extrémité large de l'entonnoir est tournée vers la face de traitement (22) ou vers la lampe (10).

22. Luminaire de traitement de thermothérapie selon l'une des revendication précédentes, **caractérisé en ce que** la pièce rapportée est remplaçable.

23. Luminaire de traitement de thermothérapie selon l'une des revendications précédentes, **caractérisé en ce que** la position de la pièce rapportée est modifiable, par coulissement ou par mouvement par vissage.

24. Luminaire de traitement de thermothérapie selon l'une des revendications 2 à 23, **caractérisé en ce que**, à l'aide de la pièce d'espacement (20), l'espacement entre l'extrémité large du réflecteur (26) et la face de traitement (22) est modifiable, dans la plage de 0,5 à 4,5 cm.

25. Luminaire de traitement de thermothérapie selon l'une des revendications précédentes, **caractérisé par** un dispositif à échelle, afin de lire la valeur de l'espacement entre la lampe (10) et la face de traitement (22).

26. Luminaire de traitement de thermothérapie selon l'une des revendications précédentes, **caractérisé par** une minuterie à contact et/ou un dispositif de mesure de température (36) dans la zone de la lampe (10), et/ou une chambre (38) pour loger une substance odoriférante ou un liquide odoriférant et/ou une fente d'aération (34) dans le boîtier (12).

27. Luminaire de traitement de thermothérapie selon l'une des revendications précédentes, **caractérisé en ce que** le dispositif d'alimentation électrique (18) comprend une ou plusieurs batteries, logée(s) dans le boîtier, ou un ou plusieurs accumulateurs logé(s) dans le boîtier et/ou un dispositif de raccordement disposé dans le boîtier (12), afin d'assurer le raccordement de la lampe (10) à une source électrique.

28. Luminaire de traitement de thermothérapie selon 27, **caractérisé en ce que** la source électrique est une source de courant à 12 Volts ou une source de courant à 220 Volts.

29. Luminaire de traitement de thermothérapie selon la revendication 28, **caractérisé en ce que** le dispositif d'alimentation électrique (18) comprend un transformateur pour la conversion de la tension de 220 Volts en une tension de 12 Volts.

30. Luminaire de traitement de thermothérapie selon la revendication 29, **caractérisé en ce que** le transformateur se trouve à l'extérieur du boîtier (12).
